# EUROPEAN PATENT APPLICATION

(11) **EP 4 497 440 A1**
(43) Date of publication of application: **29.01.2025**
(21) Application number: 24191137.9
(22) Date of filing: 26.07.2024
(51) Int. Cl.: A61K 31/575, A61P 25/00, A61P 25/28

(54) **USE OF 7-TRIFLUOROMETHYL-7ALPHA,25-DIHYDROXYCHOLESTEROL IN THE TREATMENT AND MODULATION OF AUTOIMMUNE AND INFLAMMATORY DISEASES OF THE NERVOUS SYSTEM AND NEURODEGENERATIVE DISEASES**

(30) Priority: 27.07.2023 PL 44568323
(71) Applicant: Gdanski Uniwersytet Medyczny, 80-210 Gdansk (PL)
(72) Inventor: Rutkowska, Aleksandra, 80-299 GDA SK (PL); Karaszewski, Bartosz, 80-461 Gda sk (PL)
(74) Representative: JWP Patent & Trademark Attorneys

(57) **Abstract**

The invention concerns the use of 7-trifluoromethyl-7α,25-dihydroxycholesterol (CF3-7α,25OHC) in treating and/or modulating autoimmune and/or inflammatory diseases of the nervous system and/or neurodegenerative diseases, in particular multiple sclerosis.

## Description

The invention concerns application of cholestene-7β-trifluoromethyl,3β,7α,25-triol (CF3-7α,25OHC) in treating and/or modulating autoimmune and/or inflammatory diseases of the nervous system and/or neurodegenerative diseases.

Demyelinating diseases are diseases of the nervous system where the myelin sheaths in the nervous system suffer damage and breakdown. The most recognisable demyelinating disease is multiple sclerosis (MS). MS is an autoimmune disease of the central nervous system (CNS) and is the most common cause of non-traumatic disability in young adults world-wide. It is estimated that MS affects 2.8 million people in the world, and ca. 50 thousand people in Poland, prevailingly in the age range of 20-40, with cases 2-3 times more frequent in women than men. MS causes destruction of the myelin sheaths of the nerve fibres by the immune system. Secondarily, the loss of myelin leads to degeneration of axons and death of nerve cells, but neurodegenerative processes in the disease are also primary in nature.

The number of therapies modifying the course of the disease (*disease modifying therapies,* DMT) has grown in recent years. DMTs do not treat the MS, but substantially slow down progression of the disease and improve the patients' quality of life versus the natural course of the disease. On average, they can extend the span of life which does not require assistance of others by a dozen-or-so years. The therapies act on the immune system so as to reduce the number and intensity of the flares, but have limited effect on myelin regeneration (remyelination) or protection of the nerve cells, which is most likely due to their anti-inflammatory effect instead of that primarily stimulating myelin regeneration. Moreover, studies reveal that the loss of nervous tissue in patients suffering from the MS is continuous and progresses irrespective of the received DMT therapy or the MS type. Therefore, the most pressing challenge today is to discover a therapy which would stimulate myelin regeneration. Myelin regeneration, which is a natural process at the early stages of the disease, is disturbed over time, or stops altogether. Stimulation of the processes and myelin regeneration have neuro-protective effects and can stop or reverse the progress of the disease. At the moment, there is no therapy which would increase/stimulate natural myelin regeneration processes.

Known in the state of the art is research focused on oxysterols tested for remyelination or modulation of the oligodendrocyte (cells producing myelin) biology, where the tested oxysterols do not activate or marginally activate the EBI2 receptor (GPR183). For instance, activation of the LXR (*liver x receptor*) by 25-hydroxycholesterol (25-OHC) or a synthetic particle TO901317 stimulates myelin gene expression at the level of the promotor, mRNA, and protein implying LXRα/β directly in transcriptional control of the myelin gene expression [1]. Tests in the cuprizone model, on the other hand, revealed that oxysterol 20-OHC promotes maturation of oligodendrocyte progenitor cells, to myelin-forming oligodendrocytes, thus intensifying remyelination beyond the spontaneous regeneration which takes place in the model [2].

In the course of the research works pursued by the authors of the solution, where the works continued tests on endogenous oxysterol 7α,25-OHC and its role in myelin building and/or regeneration, it was proved that the EBI2 receptor plays a major role in normal myelin development in mice [3] and that it is indispensable for remyelination in pathophysiological conditions in *ex vivo* organotypic slices of mouse cerebellum [4] and during remyelination in mice, in the cuprizone model [5].

The technical problem faced in the state of the art consists in difficulties faced when testing oxysterols due to their short metabolic stability *in vivo.* 7-hydroxylated sterols, 7α,25-OHC in particular, are metabolically unstable. Administered to mice by subcutaneous or intraperitoneal injection, 7α,25OHC is quickly eliminated from blood, its half-life being ca. 30 minutes. Another technical problem comes down to keeping the EBI2 receptor expression level during remyelination and oxysterol treatment. Yet another problem amounts to no possibility to regulate or control the expression of synthesising enzymes, especially Ch25h, Cyp7b1, and the degrading enzyme Hsd3b7 in the brain. The immune system cells arriving in too high numbers promote the demyelinating processes, which in turn results in dynamic progression of the disease and dwindling of the patient's prognosis, where in the current state of the art search continues for the compound and its administration options in diseases so that the influx of the immune system cells could be limited and controlled.

The purpose of the invention was to develop an effective therapy which would modulate the immune system using a compound stimulating remyelination, its half-life extended, which in turn would increase the chance of penetrating the central nervous system (CNS). Oxysterols, the natural ligand 7α,25OHC of the EBI2 receptor included, are metabolised fast. Unexpectedly, it turned out that when administered *in vivo* in the cuprizone model tests (on wild type mice), oxysterol(CF3-7α,25) dihydroxycholesterol (CF3-7α,25OHC) stimulates myelin regeneration and modulates the immune system reducing the number of the immune system cells in the blood and brain. In addition, it increases the EBI2 receptor expression and the synthesis of 15 lipids in the brain.

The invention concerns administration of 7-trifluoromethyl-7α,25-dihydroxycholesterol (CF3-7α,25OHC) in treating and/or modulating autoimmune and/or inflammatory diseases of the nervous system and/or neurodegenerative diseases.

Preferably, autoimmune, inflammatory diseases of the nervous system and/or neurodegenerative diseases where demyelination occurs.

Preferably, oxysterol CF3-7α,25OHC is administered to modulate the immune system and/or stimulate remyelination.

Preferably, oxysterol CF3-7α,25OHC reduces the influx of the immune system cells.

Preferably, the nervous system stands for the central nervous system or the peripheral nervous system.

Preferably, the autoimmune disease is selected from the group including: myasthenia, multiple sclerosis (MS), acute disseminated encephalomyelitis.

Preferably, the inflammatory disease of the nervous system is selected from the group including: multiple sclerosis, acute disseminated encephalomyelitis (ADEM), the Devic syndrome (NMO), chronic inflammatory demyelinating polyradiculoneuropathy (CIDP), the Guillain-Barré syndrome (GBS).

Preferably, the neurodegenerative disease is selected from the group including: the Alzheimer disease, the Parkinson disease, the Canavan disease, leukodystrophies (e.g. the Krabbe disease, adrenoleukodystrophy).

The advantage of CF3-7α,25-dihydroxycholesterol according to the invention is its *in vivo* half life extended from ca. 30 minutes to 12 hours [6], which increases its bioavailability and the chances of its penetration into the CNS, as well as achievement of a tangible therapeutic effect, namely its action modulating the immune system and stimulating myelin regeneration. In addition, the state of the art indicates that the EBI2 receptor demonstrates chemotactic properties, i.e. stimulates cell movement towards higher concentration of oxysterol 7α,25OHC. The said action of 7α,25OHC was shown on mouse and human CNS cells and lymphocytes both *in vitro* and *in vivo.* Neither the natural ligand, 7α,25OHC, nor the modified one, CF3-7α,25OHC, have been administered to date either to humans, or animals in the animal models of multiple sclerosis or any other animal models of autoimmune or inflammatory CNS diseases; hence, the effect of administering oxysterol on myelin regeneration and the immune system was unknown. The studies known in the state of the art indicated that treating mouse-cerebellar slices *ex vivo* with unmodified oxysterol inhibits the release of pro-inflammatory cytokines following chemically-induced demyelination and in this way protects against the loss of myelin being as a result of treatment with toxin (LPC). The state of the art further indicates that receptor EBI2 is engaged in normal myelination processes upon birth in the mouse and in the cuprizone model, where we have proved slower remyelination in mouse brains with a knockout of the EBI2 receptor. However, neither oxysterol CF3-7α,25OHC, nor the natural ligand 7α,25OHC were earlier used in the cuprizone model or any other animal model during remyelination. Earlier tests were conducted without the administration of oxysterols to mice with genetic deletion of the EBI2 receptor in all cells of their organisms (the so-called receptor knockout). The impact of oxysterol treatment on the number of cells of the immune system has not been tested earlier, either in cerebellum slices *ex vivo,* or *in vivo* on animals or humans.

The immunosuppressive effect is the key action mechanism of most therapies employed in treating autoimmune and inflammatory diseases such as multiple sclerosis. For instance, BHT-3009 reduces the lymphocyte T level, alemtuzumab reduces the level of the circulating T and B lymphocytes, ofatumumab is cytotoxic for B lymphocytes, rituximab and ocrelizumab reduce the level of CD20+ B lymphocytes, teriflunomide and mitoxantrone reduce proliferation of B and T lymphocytes. Modulation of the immune system, and in particular reduction of the number of circulating autoreactive lymphocytes in the blood translates directly to reduced penetration of the cells into the CNS where they propagate inflammation and destroy directly the myelin sheaths (i.e. cause demyelination). Oxysterol CF3-7α,25OHC demonstrates immunomodulating effects on the one hand, i.e. reduces the number of lymphocytes in the blood and most likely their penetration into the CNS, while on the other hand stimulates myelin regeneration acting directly in the CNS, where importantly the processes take place simultaneously in pathophysiological sense, which means that stimulation of remyelination (or elimination or inhibition of natural remyelination) does not stem solely from immunomodulating processes. This is important in that the available therapies modifying the course of the disease (DMT) demonstrated primarily the immunomodulating effects, and this means that they are verry effective in slowing down the disease (in modulating it, as the name suggests), but they do not treat it.

Scientific literature suggests that such therapies, acting both on the immune system and on the CNS cells directly, may be effective in treating autoimmune, inflammatory, and demyelinating diseases such as multiple sclerosis. Moreover, the tests on mice conducted by the authors reveal that oxysterol CF3-7a,25-OHC reduces the level of pro-inflammatory cytokines in the CNS and of lymphocytes in blood, hence to an extent imitates the action of some DMTs, e.g. fingolimod (Gilenya, pFTY720). In view of its properties, once preclinical and clinical tests have been successfully completed, the modified (i.e. of extended half-life in vivo) oxysterol CF3-7α,25-OHC may become the first therapy modulating myelin regeneration in the CNS and come to be used as therapy for multiple sclerosis or other demyelinating diseases in Poland and the world, where modified oxysterol denotes the derivative which contains appropriate alkyl substituent in the 7^{th} position which creates a more stable, tertiary allyl hydroxyl group making oxysterol resistant to oxidation and metabolic degradation.

The object of the invention is presented on figures of the drawing, where:

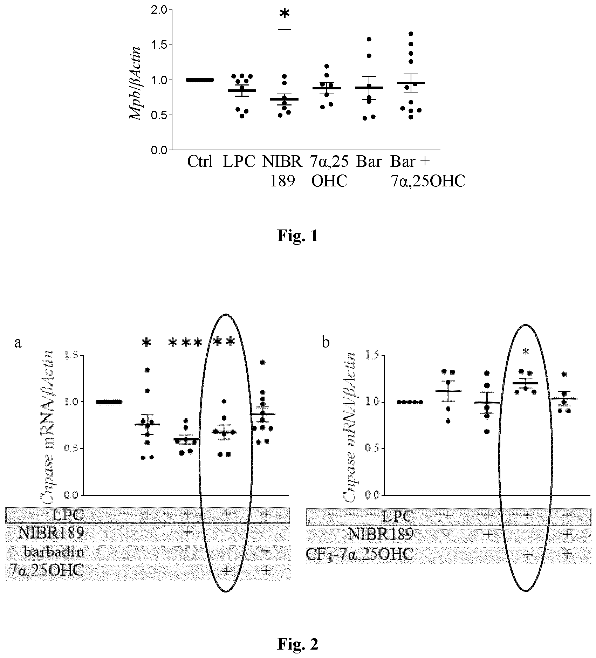

- Fig. 1: presents quantification of mRNA of the *Mbp* gene (*myelin basic protein*) in mouse organotypic cerebellar slices whose myelin was first chemically removed with lysophosphatidylcholine (LPC) and which were then treated with 7α,25OHC, barbadin (bar), or an antagonist of the EBI2 receptor, NIBR189. The slices treated with the antagonist do not remyelinate, whereas slices treated simultaneously with barbadin and non-modified oxysterol (Bar+7α,25OHC) remyelinate faster (the highest *Mbp* expression). The slices treated only with non-modified oxysterol 7α,25OHC do not remyelinate quicker than ones not subject to any treatment.
- Fig. 2: presents the differences in the action of the natural ligand of receptor EBI2, 7α,25OHC (a), and of the modified one, CF3-7α,25OHC (b). Organotypic cerebellar slices treated with the natural ligand do not remyelinate (regenerate the myelin) quicker than slices not treated with anything at all (LPC group) (first graph). The slices treated with CF3-7α,25OHC on the other hand, remyelinate quicker than slices not treated with anything (second graph). Barbadin, which inhibits internalisations of receptor EBI2, is needed to attain the therapeutic effect (accelerated remyelination) of non-modified oxysterol. Barbadin is not needed for modified oxysterol, CF3-7α,25OHC.

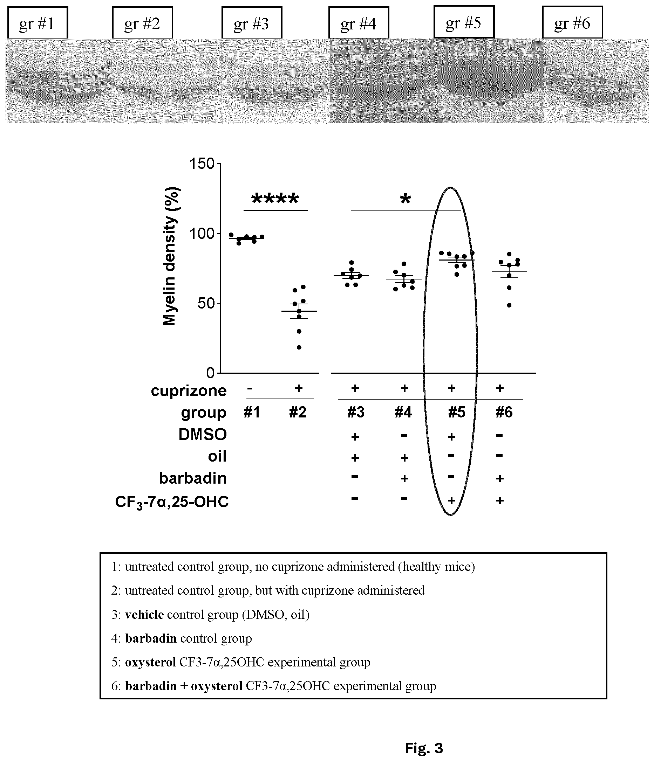

- Fig. 3: presents **mechanism 1**: luxol-stained sections of mouse brain from the cuprizone model and quantification of the myelin density. The density of myelin in the corpus callosum increased only in mice injected with CF3-7α,25OHC, group #5, compared to the control group #3 which was treated with vehicle (DMSO or canola oil) where myelin regeneration develops autonomously. The data show that extension of bioavailability of oxysterol (= use of CF3-7α,25OHC) is sufficient to attain the therapeutic effect (accelerated remyelination) which makes barbadin redundant.

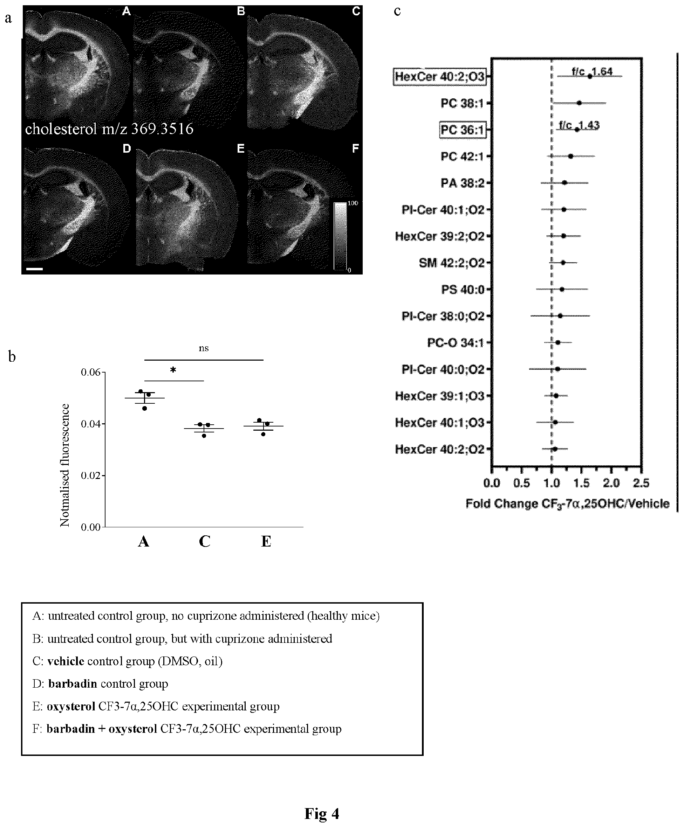

- Fig. 4: presents **mechanism 1**: mouse brains from the cuprizone model, as above a) images of cholesterol in mouse brains, taken with mass spectrometer, and b) graph visualizing cholesterol quantification in the central part of the corpus callosum in groups: A (control, no cuprizone), C (control, following cuprizone administration, not given any treatment), and E (treated with CF3-7α,25OHC). c) Therapy with CF3-7α,25OHC increases concentration of 15 lipids in the brain (group E) compared to untreated animals (group C), where: PC: phosphatidylcholine; PS: phosphatidylserine; MS: sphingomyelin; PE: phosphatidylethanolamine; PA: phosphatidic acid; GlcCer: glucosylceramide; GalCer: galactosylceramide; PI-Cer: ceramide phosphoinositol. The data show that increased EBI2 signalling obtained by activation with CF3-7α,25OHC increases lipid synthesis in the brain (myelin is built of lipids).

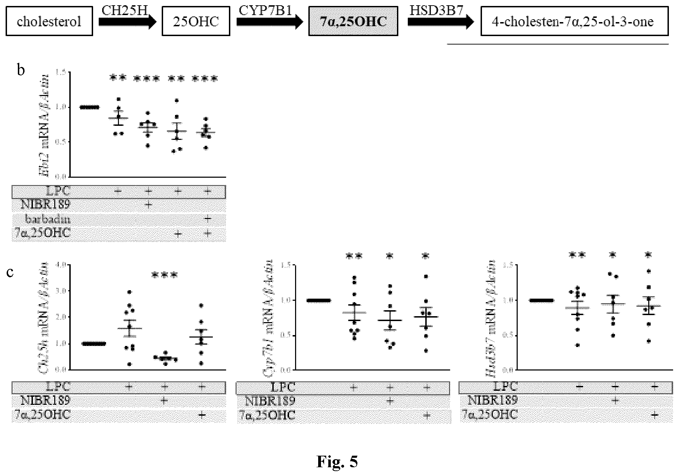

- Fig. 5: presents **mechanism 2,** where natural ligand 7α,25OHC reduces the expression level of receptor EBI2 and regulates the expression level of the enzymes synthesizing and degrading the natural ligand 7α,25OHC in organotypic cerebellum slices, where: a) presents synthesis of 7α,25OHC, b) presents quantification of EBI2 in the brain after demyelination with LPC, treatment with 7α,25OHC does not increase its expression, and c) treatment with LPC regulates enzymes synthesising and degrading natural oxysterol 7α,25OHC.Treatment with 7α,25OHC, in addition, reduces the level of *Ch25h.*

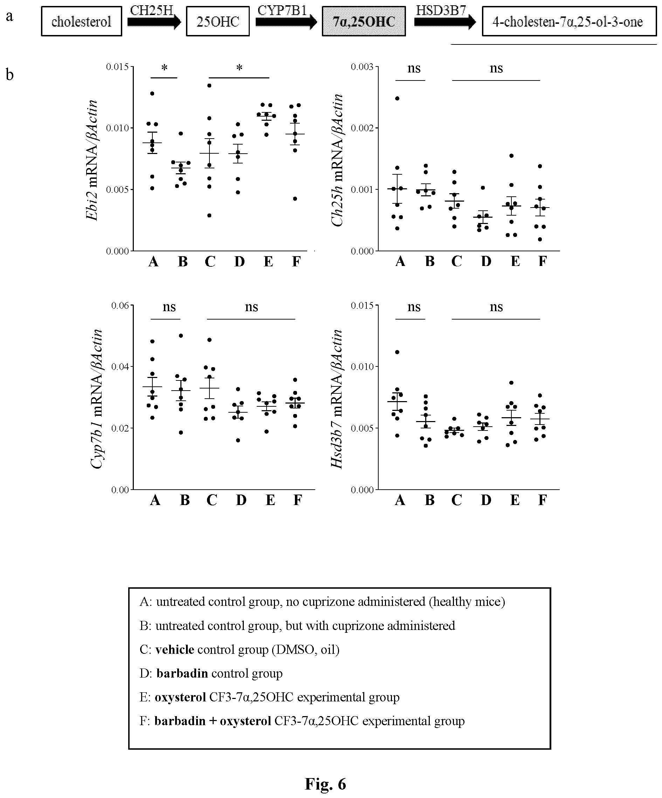

- Fig. 6: presents **mechanism 2,** where modified oxysterol CF3-7α,25OHC increases the EBI2 expression level during remyelination in the cuprizone model, but does not regulate the expression level of the synthesizing enzymes (*Ch25h, Cyp7b1*) or the degrading one *(Hsd3b7)* in the cerebellum in the cuprizone model, where: a) presents synthesis of 7α,25OHC, b) presents quantification of EBI2 and enzymes in the cerebellum in the cuprizone model. Expression of EBI2 increased only in mice treated with CF3-7α,25OHC.The enzyme level remained unchanged, which points to action different from the unmodified ligand 7α,25OHC measured in organotypic cerebellar slices (Fig 5).

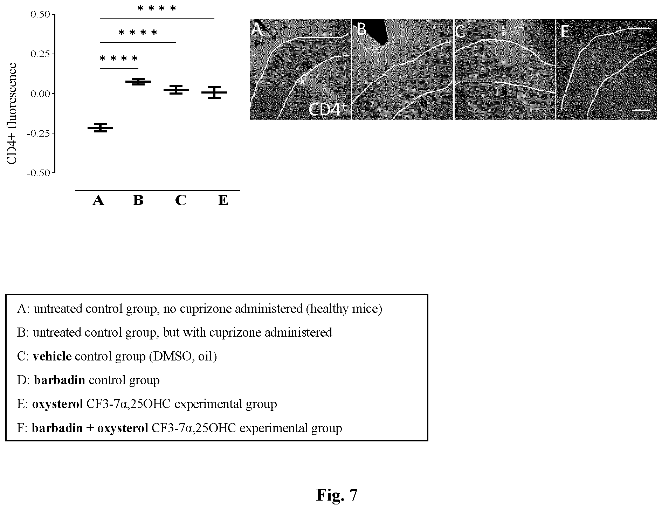

- Fig. 7: presents quantitative determination of immunofluorescently tagged CD4+ cells in the central part of the corpus callosum after 14 days of treatment in the cuprizone model. The fewest CD4+ cells are found in the corpus callosum of mice treated with CF3-7α,25OHC.The data indicate that the mechanism of action of CF3-7α,25OHC may be limiting the inflow of the immune system cells to the brain, thanks to which the myelin destruction by the cells is restricted.

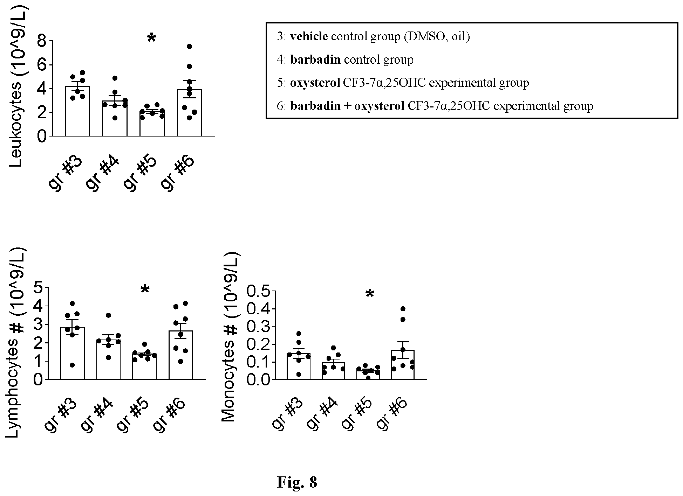

- Fig. 8: presents **mechanism 3,** where CF3-7α,25OHC reduces the number of white blood cells. The graphs present a 51% drop in the total number of white blood cells, a 14% drop in neutrophils, a 51% drop in lymphocytes, a 66% drop in monocytes, a 43% drop in eosinophils after 14 days of treatment during remyelination in the cuprizone model. The drop in the count of the immune system cells is recorded only for group E (=group #5) i.e. the one treated only with modified oxysterol CF3-7α,25OHC. The data show the action mechanism of CF3-7α,25OHC, namely reduction of the number of white blood cells, which carries therapeutic effect since the cells destroy the myelin and escalate inflammation.

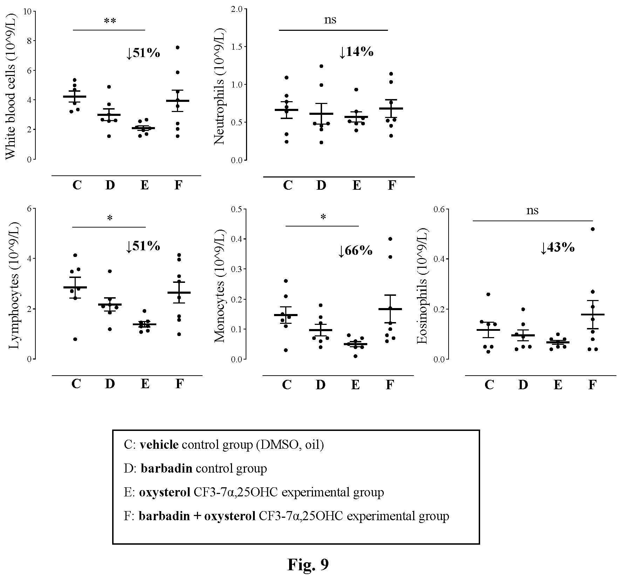

- Fig. 9: presents the mouse blood morphology results after 9 weeks of cuprizone diet, then treated, respectively, with vehicles (dimethyl sulfoxide (DMSO) or canola oil) - group #3, barbadin - group #4, or CF3-7α,25OHC - group #5, or simultaneously with barbadin and CF3-7α,25OHC - group #6. The number of white blood cells went down only in mice which were injected with CF3-7α,25OHC (group #5). The data show the mechanism of action of CF3-7α,25OHC, namely reduction of the number of white blood cells, which carries therapeutic effect since the cells destroy the myelin and escalate inflammation.

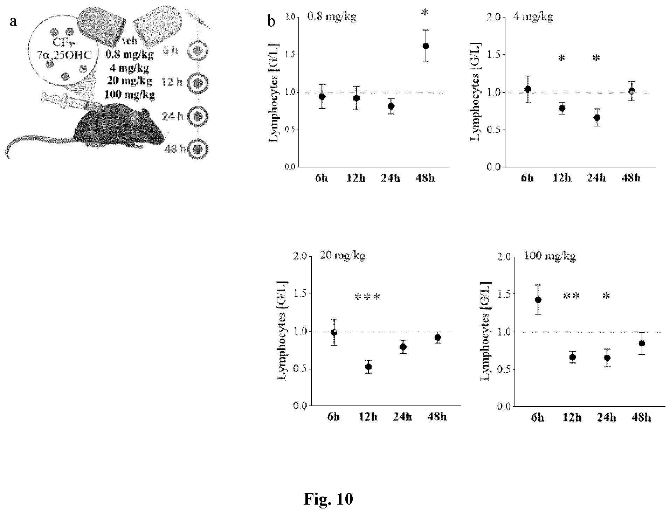

- Fig. 10: presents **mechanism 3,** where CF3-7α,25OHC reduces the number of white blood cells, and where a) presents configuration of a new, short-term pharmacokinetic experiment consisting of injection with 4 doses of CF3-7α,25OHC (0.8 mg/kg, 4 mg/kg, 20 mg/kg, 100 mg/kg, or vehicle (canola oil = control) for 6, 12, 24, or 48 hours. Then, blood count was taken. b) CF3-7α,25OHC reduces the level of lymphocytes with the dose as low as 4 mg/kg and the effect continues for 24 hours following administration. The green line shows the number of lymphocytes in the control group.

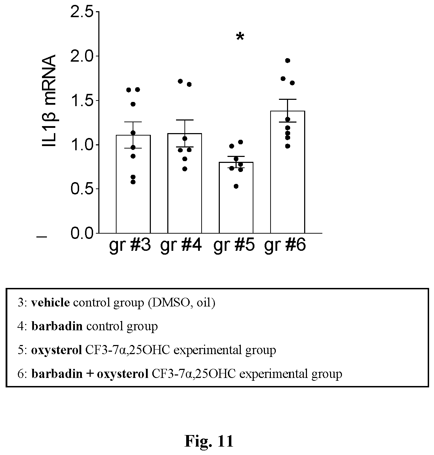

- Fig. 11: presents quantification of pro-inflammatory cytokine IL1β in cerebellum lysates of mice of the cuprizone model; mice groups as in fig. 3. Reduction of the number of pro-inflammatory cytokines was observed only in mice injected with CF3-7α,25OHC (group #5).

The invention is illustrated with the following examples.

Used in the examples were:
- The 7α,25OHC compound by Novartis, Basel (gift donated in cooperation upon signing of the MTA). The molecule commercially available, e.g. Merck cat. No. 128136.
- The CF3-7α,25OHC compound synthesized by a chemist from the Gdańsk University of Technology from a starting molecule of 25-OHC (Chemat cat. No. AG0037V4-250mg) in accordance with the procedure described in publication: Deng et al., 2016 [6].
- NIBR189 ((2E)-3-(4-bromophenyl)-1-(4-(4-methoxybenzyoil)-1-piperazine)-2-propen-1-on, (E)-3-(4-bromophenyl)-1-(4-(4-methoxybenzyoil)piperazine-1-ylo)prop-2-en-1-on) by Novartis, Basel (a gift donated in cooperation upon signing of the MTA). The molecule commercially available, e.g. Merck cat. No. SML1981.
- lysophosphatidylcholine (LPC): Merck cat. No. L4129-25mg.
- barbadin: Merck cat. No. SML3127-5MG.
- vehicles (canola oil, dimethyl sulfoxide (DMSO): Merck cat. No. D8418-50ML).
- chow with cuprizone and control chow (cuprizone-free): Vivari (produced on commission, S8435-E080 EF AIN93G), cuprizone content: 0.2%.

### Example 1

**Organotypic cerebellar slices** were sampled from ten-day old mice and then grown in an incubator for 14 days. After 14 days the myelin was chemically removed with LPC (0.4 mg/ml). Following the removal of the myelin sheaths, the slices were treated with:
1. the control group: regular culture medium, without LPC
2. LPC, then on serum-free culture medium for 14-16 days
3. LPC followed by barbadin 16 µM in serum-free culture medium for 14-16 days
4. LPC followed by 7α,25OHC 0.1 µM in serum-free culture medium for 14-16 days
5. LPC followed by NIBR189 10 µM in serum-free culture medium for 14-16 days
6. LPC followed by barbadin 16 µM + 7α,25OHC 0.1 µM in serum-free culture medium for 14-16 days

Once the experiment was completed (remyelination for 14-16 days) the slices were collected and MBP mRNA levels were measured by the PCR quantitative method. In the mouse organotypic cerebellar slices in which myelin sheaths were chemically removed with lysophosphatidylcholine (LPC) spontaneous myelin regeneration took place except for the slices treated with a selective EBI2 receptor antagonist (NIBR189). The results once again reveal key significance of EBI2 intracellular signalling for myelin regeneration. The slices treated with the antagonist (NIRB189) do not remyelinate, while the slices treated with both barbadin and non-modified oxysterol (Bar+7α,25OHC) remyelinate the fastest (the highest expression of *Mbp and Cnpase,* Fig 1 and Fig 2a). The slices treated only with non-modified oxysterol 7α,25OHC do not remyelinate faster than the untreated slices, which indicates that treatment with non-modified oxysterol alone is insufficient to achieve therapeutic effects, i.e. higher remyelination. On the other hand, the slices treated with modified oxysterol CF3-7α,25OHC remyelinate faster than the untreated slices (second graph of Fig 2b). Barbadin, which inhibits internalisation of the EBI2 receptor, is necessary to achieve the therapeutic effect (faster remyelination) in the case of non-modified oxysterol (Fig 1 and Fig 2a). Barbadin is not required in the case of modified oxysterol, CF3-7α,25OHC (Fig 2b), which indicates that the action of the modified compound differs from that of the natural one.

### Example 2

Measurement of lipoprotein density in myelin sheaths, taken on sections of brains of the mice in the **cuprizone model.** The mice in the cuprizone model subject were divided into 6 groups:
**#1. control group:** regular **cuprizone-free** chow for 9 weeks. The mice were euthanised after 9 weeks and tissue samples were taken for analysis. In these mice no removal of myelin sheaths occurred.
**#2. control group with cuprizone:** chow **containing cuprizone** for 9 weeks. The mice were euthanised after 9 weeks and tissue samples were taken for analysis. In the mice fed with chow containing cuprizone myelin sheaths were removed.
**#3. vehicle control group** - the mice were fed with chow containing cuprizone for 9 weeks, and then with regular chow without cuprizone for 2 weeks. Over the 2 weeks on regular show the mice in the vehicle control group were first injected with vehicle #1: DMSO, intraperitoneally, 150 µl once a day, and then with vehicle #2: canola oil, subcutaneously, 150 µl once a day.
**#4. barbadin control group** - the mice were fed with chow containing cuprizone for 9 weeks and then with regular chow without cuprizone for 2 weeks. Over the 2 weeks on regular chow the mice in this group were injected with barbadin 0.3 mg/kg, intraperitoneally, 150 µl once a day and then with vehicle #2: canola oil, subcutaneously, 150 µl once a day.
**#5. oxysterol experimental group** - the mice were fed with chow containing cuprizone for 9 weeks and then with regular chow without cuprizone for 2 weeks. Over the 2 weeks on regular chow the mice in this group were injected with vehicle #1: DMSO, intraperitoneally, 150 µl once a day, and with CF3-7α,25OHC, dose: 20 mg/kg, subcutaneously, 150 µl once a day.
**#6 barbadin** + **oxysterol experimental group** - the mice were fed with chow containing cuprizone for 9 weeks and then with regular chow without cuprizone for 2 weeks. Over the 2 weeks on regular chow the mice in this group were injected with barbadin 0.3 mg/kg, intraperitoneally, 150 µl once a day, and then after one hour following the barbadin injection with CF3-7α,25OHC, dose: 20 mg/kg, subcutaneously, 150 µl once a day.

Brain sections sampled from the mice were histochemically stained with luxol fast blue (Abcam, cat. No. Ab 150675), and the intensity (density) of the lipid staining in the corpus callosum was measured using the ImageJ software (ImageJ (nih.gov)).

The measurement of lipoprotein density in myelin sheaths in the corpus callosum using the luxol fast blue stain revealed more efficient (or faster) myelin regeneration in the corpus callosum of mice treated with CF3-7α25OHC (group #5) compared to the untreated mice (group #3), in which the myelin regeneration is a natural process. The lipoprotein density in the corpus callosum of the mice injected with CF3-7α25OHC (group #5) was higher by 16% compared to the density in the corpus callosum of the mice of group 3# injected with the same vehicles, in which myelin regeneration is a natural process.

Mouse brain sections were imaged using a mass spectrometer and then the lipid density was quantified (Fig 4). The content of cholesterol, i.e. the main myelin component, in the corpus callosum was higher following the therapy with modified oxysterol CF3-7a25OHC compared to the control group treated with vehicles, in which the myelin regenerates spontaneously. Moreover, synthesis of 15 lipids in the corpus callosum was higher in the group treated with CF3-7a25OHC.

The demonstrated effects of CF3-7α25OHC which consist in boosting the effectiveness of myelin sheath regeneration are of crucial significance in treating demyelinating diseases such as multiple sclerosis. There are a dozen-or-so existing therapies which modify the course of the MS. The therapies do not cure the MS definitely, but slow down the progress of the disease substantially and improve the quality of the patients' lives. The modifying therapies primarily demonstrate action on the immune system by cutting down the number of the immune cells penetrating into the CNS, thus reducing inflammation, the number of pro-inflammatory cytokines, etc. Their effects on the CNS cells and neurodegenerative or neuroprotective processes, on the other hand, are insignificant. Research shows that the loss of nervous tissue in patients suffering from MS is a continuous process which occurs irrespective of the therapy received or the MS type. That is why the most pressing challenge faced today is to discover a therapy which would stimulate myelin regeneration. Myelin regeneration, which continues to occur as a natural process in early stages of the disease, gets disrupted or stops entirely over time. Stimulation of the processes and myelin regeneration carries neuroprotective effects and can stop or entirely reverse the progress of the disease. Today, there are no therapies which would boost/ stimulate the natural processes of myelin regeneration. Oxysterol CF3-7α,25OHC can be the first therapy modulating myelin regeneration in the CNS and can be used as therapy to treat MS or other demyelinating diseases in Poland and the world.

### Example 3

Measurement of the mRNA level of the *Ebi2* receptor and of the enzymes synthesizing (*Ch25h, Cyp7b1*) and degrading *(Hsd3b7)* the natural ligand 7α,25OHC **in organotypic cerebellar slices** treated with non-modified oxysterol 7α,25OHC (Fig 5) (tissue and treatments as in Fig 1 and Fig 2a) **and in cerebella of the mice subject to the cuprizone model,** treated with modified oxysterol CF3-7α,25OHC (Fig 6). LPC treatment modulates enzymes which synthesise and degrade the natural oxysterol 7α,25OHC. In addition, treatment with 7α,25OHC reduces the level of *Ch25h.* Treatment of slices first treated with LPC and then with 7α,25OHC does not increase *Ebi2* expression. Modified oxysterol CF3-7α,25OHC, on the other hand, increases the level of *Ebi2* expression during remyelination in the cuprizone model but does not regulate the expression level of the synthesizing (*Ch25h, Cyp7b1*) and degrading *(Hsd3b7)* enzymes in the cerebellum. These data reveal that the action of non-modified and modified oxysterol is different and show that the observed therapeutic action (mechanism of action) is modulated by and dependent on intracellular signaling of the EBI2 receptor activated by its ligand/agonist, oxysterol.

### Example 4

Measurement of the quantity of immunofluorescently-tagged CD4+ cells in the central part of the corpus callosum following 14 days of treatment in the cuprizone model (Fig 7). Morphology of mouse blood in the cuprizone model and the *in vivo* model testing short-term pharmacotherapy of oxysterol CF3-7α,25OHC (Fig 10).

A (Fig 7) **control group:** regular **cuprizone-free** chow for 9 weeks. Mice were euthanised after 9 weeks and tissue samples were taken for analysis. In these mice, myelin sheaths were not removed.

**B** (Fig 7) **control group with cuprizone:** chow **containing cuprizone** for 9 weeks. The mice were euthanised following 9 weeks and tissue samples were taken for analysis. In the mice fed with cuprizone-containing chow myelin sheaths were removed.

**C** (Fig 7) **or #3** (Fig 8 and 9) **vehicle control group** - the mice were fed with chow containing cuprizone for 9 weeks, and then with regular chow without cuprizone for another 2 weeks. Over the 2 weeks on regular chow the mice in the vehicle control group were first injected vehicle #1: DMSO (intraperitoneal injection), 150 µl once a day, and then vehicle #2: canola oil, subcutaneously, 150 µl once a day.

**#4** (Fig 8 and 9) **barbadin control group** - the mice were fed with chow containing cuprizone for 9 weeks, and then with regular chow without cuprizone for another 2 weeks. Over the 2 weeks on regular chow the mice in the group were injected barbadin 0.3 mg/kg, intraperitoneally, 150 µl once a day, and then vehicle #2: canola oil, subcutaneously, 150 µl once a day.

**E** (Fig 7) **or #5 experimental group with oxysterol CF3-7α,25OHC** - the mice were fed with chow containing cuprizone for 9 weeks, and then with regular chow without cuprizone for another 2 weeks. Over the 2 weeks on regular chow the mice in the group were injected vehicle #1: DMSO, intraperitoneally, 150 µl once a day, and CF3-7α,25OHC, dose: 20 mg/kg, subcutaneously, 150 µl once a day.

**#6** (Fig 8 and 9) **experimental group with barbadin + oxysterol** CF3-7α,25OHC - the mice were fed with chow containing cuprizone for 9 weeks, and then with regular chow without cuprizone for another 2 weeks. Over the 2 weeks on regular chow the mice in the group were injected barbadin, 0.3 mg/kg, intraperitoneally, 150 µl once a day, and then one hour following the barbadin injection they were injected CF3-7α,25OHC, dose: 20 mg/kg, subcutaneously, 150 µl once a day.

Mouse groups in the short-term pharmacology model (Fig 10):
**Control:** one vehicle injection (canola oil). The number of lymphocytes in the group is marked on the graph with the transverse line. The vehicle injection was administered to the mice subcutaneously (100 µl), following which the mice were euthanized after 6 hours (8 mice), 12 hours (8 mice), 24 hours (8 mice), or 48 hours (8 mice).

**The 0.8 mg/kg dose group:** single injection with CF3-7α,25OHC at the dose of 0.8 mg/kg. The oxysterol injection was administered to the mice subcutaneously (100 µl), following which the mice were euthanized after 6 hours (8 mice), 12 hours (8 mice), 24 hours (8 mice), or 48 hours (8 mice).

**The 4 mg/kg dose group:** single injection with CF3-7α,25OHC at the dose of 4 mg/kg. The oxysterol injection was administered to the mice subcutaneously (100 µl), following which the mice were euthanized after 6 hours (8 mice), 12 hours (8 mice), 24 hours (8 mice), or 48 hours (8 mice).

**The 20 mg/kg dose group:** a single injection with CF3-7α,25OHC at the dose of 20 mg/kg. The oxysterol injection was administered to the mice subcutaneously (100 µl), following which the mice were euthanized after 6 hours (8 mice), 12 hours (8 mice), 24 hours (8 mice), or 48 hours (8 mice).

**The 100 mg/kg dose group:** a single injection with CF3-7α,25OHC at the dose of 20 mg/kg. The oxysterol injection was administered to the mice subcutaneously (100 µl), following which the mice were euthanized after 6 hours (8 mice), 12 hours (8 mice), 24 hours (8 mice), or 48 hours (8 mice).

The encephala for CD4+ staining were sampled immediately after the mice had been euthanised, and were soaked in paraformaldehyde 4% for 24 hours, and then in sucrose solutions. The encephala were cut into sections on a cryostat and immunohistochemically stained using anti-CD4+ antibodies (ThermoFisher, catalogue No. BS-0647R). The lowest count of the CD4+ cells was found in the corpus callosum of the mice treated with CF3-7α,25OHC (group E). These data show that the action mechanism of CF3-7α,25OHC can consist in reducing the inflow of the immune system cells to the brain, thanks to which myelin destruction caused by these cells is reduced.

The blood from the heart was sampled after the mice had been euthanised, and blood morphology test was performed using a standard diagnostic analyser. The tests revealed a substantially reduced count of leukocytes, lymphocytes, and monocytes in the mice treated with oxysterol CF3-7α,25OHC (gr #5 or E). The lymphocyte count in peripheral blood of the mice which had been injected with CF3-7α,25OHC was lower by 50% compared to the control group of mice injected with vehicles (gr. #3 or C). The specific action of CF3-7α,25OHC leading to reduction of the lymphocyte count in blood finds confirmation in the results obtained for the mice of groups #4 (or D) and #6 (or F). Compared to the control group, the lymphocyte count in blood in the group of mice injected with barbadin (#4 or D) was lower only by 21%, and in the group of mice injected with barbadin + CF3-7α,25OHC by 7%. Following interaction with CF3-7α,25OHC, barbadin blocks internalisation of the EBI2 receptor from the cell membrane. The absence of any significant effect related to the treatment with barbadin or barbadin + CF3-7α,25OHC indicates a specific action of CF3-7α,25OHC on the EBI2 receptor and the consequential effects of that action, namely reduction of the count of circulating lymphocytes in blood. Moreover, the number of monocytes in group #5 (or E: mice treated with CF3-7α,25OHC) was lower by as much as 66% and the total leukocyte count (lymphocytes and monocytes included) was also substantially lower (by 51%), though only in the group of mice injected with CF3-7α,25OHC. In our subsequent *in vivo* tests (short-term pharmacology) we confirmed the action of CF3-7α,25OHC. In that test, too, it reduced the count of white blood cells. CF3-7α,25OHC reduces the lymphocyte count as early as following the 4 mg/kg dose and the effect lasts for 24 hours following administration.

The action of CF3-7α,25OHC consisting in the reduction of the number of circulating lymphocytes is of immense significance in therapy of autoimmune diseases, such is e.g. multiple sclerosis, because in multiple sclerosis the autoreactive cells (i.e. those reacting to the proteins present in the organism) of the immune system, lymphocytes in particular, penetrate into the CNS where they propagate inflammation and destroy the myelin sheaths of the nerve fibres (demyelination). The therapeutic effect of treatment which modulates the course of multiple sclerosis consists in blocking the penetration of lymphocytes into the CNS, or in reducing their count in blood. Since similar effects were observed in patients suffering from MS following two weeks of therapy with fingolimod (pFTY720, Gilenya) (their total lymphocyte count dropped by 70%[7]), it can be concluded that oxysterol CF3-7α,25OHC can find similar application.

### Example 5

Identifying the mRNA levels of pro-inflammatory cytokine IL1β in mouse cerebellum lysates (Fig 11). The mice of groups #3, #4, #5 and #6 were described in detail in examples 2 and 4.

An analysis of the mRNA levels of pro-inflammatory cytokine IL1β in mouse cerebellum lysates, performed by the PCR method revealed the mRNA level dropped by 28% in the group treated with CF3-7a25OHC (#5) compared to the group treated solely with vehicles, which testifies to anti-inflammatory effects in the CNS. The mRNA level of the IL1β cytokine went up by 2% in the cerebella of the mice treated with barbadin, and by 25% in the group treated with barbadin + CF3-7α25OHC versus the group treated solely with vehicles. The results demonstrate that CF3-7a25OHC penetrates the blood-brain barrier and passes into the brain, and that it demonstrates anti-inflammatory action in the CNS directly. This action is of key significance in the context of treating multiple sclerosis. The inflammation which occurs in this autoimmune inflammatory disease of the CNS leads to destruction of the myelin sheaths of the nerve fibres by the auto-reactive cells of the immune system. Pro-inflammatory cytokine IL1β is one of the main cytokines the level of which increases in multiple sclerosis and which propagates inflammation and activation of the immune system cells. Reducing the level of this pro-inflammatory cytokine in blood and brain carries therapeutic effects in the treatment of autoimmune and inflammatory diseases, such as multiple sclerosis.

### BIBLIOGRAPHY

1. Meffre D, Shackleford G, Hichor M, et al. Liver X receptors alpha and beta promote myelination and remyelination in the cerebellum. Proc Natl Acad Sci U S A. 2015;112(24):7587-7592. doi:10.1073/PNAS.1424951112
2. ECTRIMS 2022 - Poster. https://doi.org/101177/13524585221123687. 2022;28(3_suppl):130-691. doi:10.1177/13524585221123687
3. Rutkowska A, Sailer AW, Dev KK. EBI2 receptor regulates myelin development and inhibits LPC-induced demyelination. J Neuroinflammation. 2017;14(1):250. doi: 10.1186/s12974-017-1025-0
4. Velasco-Estevez M, Koch N, Klejbor I, et al. Ebi2 is temporarily upregulated in mo3.13 oligodendrocytes during maturation and regulates remyelination in the organotypic cerebellar slice model. Int J Mol Sci. 2021;22(9). doi:10.3390/ijms22094342
5. Klejbor I, Shimshek DR, Klimaszewska-Lata J, et al. EBI2 is expressed in glial cells in multiple sclerosis lesions, and its knock-out modulates remyelination in the cuprizone model. Eur J Neurosci. 2021;54(3):5173-5188. doi:10.1111/EJN.15359
6. Deng X, Sun S, Wu J, et al. Fluoro analogs of bioactive oxy-sterols: Synthesis of an EBI2 agonist with enhanced metabolic stability. Bioorg Med Chem Lett. 2016;26(20):4888-4891. doi:10.1016/j.bmcl.2016.09.029
7. Fox EJ, Lublin FD, Wolinsky JS, et al. Lymphocyte counts and infection rates: Long-term fingolimod treatment in primary progressive MS. Neurol Neuroimmunol Neuroinflammation. 2019;6(6). doi:10.1212/NXI.0000000000000614

## Claims

1. 7-trifluoromethyl-7α,25-dihydroxycholesterol (CF3-7α,25OHC) for application in treating and/or modulating autoimmune and/or inflammatory diseases of the nervous system and/or neurodegenerative diseases.

2. Oxysterol CF3-7α,25OHC for application in accordance with Claim 1, where the autoimmune, inflammatory diseases of the nervous system, and/or neurodegenerative diseases are connected with demyelination.

3. Oxysterol CF3-7α,25OHC for application in accordance with any of Claims 1-2, where it is used to modulate the immune system and/or stimulate remyelination.

4. Oxysterol CF3-7α,25OHC for application in accordance with any of Claims 1-3, where it reduces the inflow of the immune system cells.

5. Oxysterol CF3-7α,25OHC for application in accordance with Claim 1, where the nervous system denotes the central nervous system and the peripheral nervous system.

6. Oxysterol CF3-7α,25OHC for application in accordance with Claim 1, where the autoimmune disease is any of the group comprising: myasthenia, multiple sclerosis, acute disseminated encephalomyelitis.

7. Oxysterol CF3-7α,25OHC for application in accordance with Claim 1, where the inflammatory disease of the nervous system is any of the group comprising: multiple sclerosis, acute disseminated encephalomyelitis (ADEM), Devic's disease (NMO), chronic inflammatory demyelinating polyradiculoneuropathy (CIDP), Guillain-Barré syndrome (GBS),

8. Oxysterol CF3-7α,25OHC for application in accordance with Claim 1, where the neurodegenerative disease is any of the group comprising: Alzheimer's disease, Parkinson's disease, Canavan disease, leukodystrophies (e.g. Krabbe disease, adrenoleukodystrophy).
